# EUROPEAN PATENT SPECIFICATION

(11) **EP 2 465 581 B1**
(45) Date of publication and mention of the grant of the patent: **05.02.2020**
(21) Application number: 10195263.8
(22) Date of filing: 15.12.2010
(51) Int. Cl.: A61Q 7/00, A61K 8/49

(54) **Non-therapeutic use of a composition for promoting hair growth**
Nicht-therapeutische Verwendung einer Zusammensetzung zur Unterstützung des Haarwachstums
Utilisation non-thérapeutique d'une composition permettant de favoriser la croissance des cheveux

(43) Date of publication of application: 20.06.2012
(73) Proprietor: Energenesis Biomedical Co., Ltd., New Taipei City 235 (TW)
(72) Inventor: Chen, Han-Min, Da'an District Taipei City 106 (TW); Chin, Li-Te, North District Hsinchu City (TW)
(74) Representative: Epping - Hermann - Fischer

(56) References cited:
- EP-A1- 0 107 885
- EP-A1- 1 557 159
- EP-A1- 2 174 647
- WO-A1-96/25943
- WO-A1-2008/028773
- WO-A2-2008/132101
- DE-A1- 4 323 616
- DE-A1- 10 120 606
- DE-A1-102006 010 870
- FR-A1- 2 620 024
- US-A1- 2003 157 176
- US-A1- 2005 000 040

## Description

### BACKGROUND OF THE INVENTION

### 1. Technical Field

The present invention is an invention belonging to the life style improving cosmetic in the field of hair maintenance and enrichment. More particularly, it relates to a non-therapeutic use of a composition for promoting hair growth, a method of promoting eyelash and eyebrow growth, and a non-therapeutic use of a composition for preventing hair fall.

### 2. Description of the Related Art

As the aged population is increasing and stress is high in modern society, there are a growing number of people under the threat of hair fall and hair loss. Although medically benign, they can cause tremendous emotional and psychosocial pressure in affected patients and their families. The vast majority of people are thus trying to avoid baldness or alopecia like the plaque, thus an effective treatment is urgently needed.

### The Hair Growth Cycle

In mammals, hair does not continuously grow but undergoes a cycle of activity involving alternate periods of growth and rest. Three stages in the hair growth cycle are as follows,
(i) Anagen: an active stage, during which the hair follicle penetrates deep into the dermis with the cells of the bulb dividing rapidly and differentiating to form the hair,
(ii) Catagen: a regressive stage, during which the follicle regresses upwards through the dermis and hair growth ceases,
(iii) Telogen: a resting stage, in which the regressed follicle contains a small secondary germ with an underlying ball of tightly packed dermal papilla cells.

The initiation of a new anagen stage is revealed by rapid proliferation in the germ, expansion of the dermal papilla and elaboration of basement membrane components. The hair cycle is then repeated many times until, as a consequence of the onset of male pattern baldness, most of the hair follicles spend an increasing proportion of their time in the telogen stage, and the hairs produced become finer, shorter, and less visible; this is known as terminal to vellus transformation.

It is generally considered that hair loss is elicited by male hormones, dihydrotesterone (DHT), produced in the hair follicles. DHT may decrease the flow of blood to the hair papilla and hair follicles. It may also cause hypersteatosis, abnormalities on the scalp due to production of reactive oxygen species and/or poor nutrition. Conventional hair tonic compositions have thus been formulated with ingredients having actions in eliminating or alleviating these causes. For example, in order to improve the circulation of blood at the scalp, vasodilators such as swertia herb extract, vitamin E and its derivatives, acetylcholine derivatives, and skin function promoters such as cepharanthine were formulated. Antipyrotics such as shikon extract were also formulated to suppress inflammation of the scalp. Furthermore, to enrich the hair follicles, amino acids such as serine and methione, vitamins such as vitamin B 6, are formulated. These are used for the purpose of the prevention of hair loss, the promotion of hair growth.

Two medical treatments were approved by the U.S. Food and Drug Administration (FDA) for male baldness, finasteride (Johannsson, U.S. Pat. No. US 2006/0099251 A1) and minoxidil (Chidsey, U.S. Pat. No. 4,139,619). Finasteride was originally used for the treatment of benigh prostatic hyperplasia (BPH) and subsequently found effective for the treatment of male pattern baldness . Finasteride works by inhibiting the activity of 5-alpha-reductase, the enzyme responsible for the conversion of free testosterone to DHT that may induce the follicular miniaturization (Dallob et al, 1994). Finasteride was now marketed as the brand-name drugs, Propecia, by Merck for the baldness treatment on men but not women. Minoxidil is a vasodilator and was originally used as an oral drug (Loniten) to treat high blood pressure. It was discovered to have the side effect of hair growth and reversing baldness in the 1980s. Upjohn Corporation received FDA approval to market a topical solution that contained 2% minoxidil to be used to treat baldness and hair loss (Rogaine and Regaine). The acting mechanism for minoxidil is unclear, but many speculate that by widening blood vessels, opening potassium channels, and allowing more oxygen, blood and nutrients to the follicle.

Minoxidil-induced hair growth was also suggested mediated by adenosine (Li et al, 2001), and presumably through the A2b adenosine receptor pathways (Iino et al, 2007) . Therefore, adenosine was unveiled as another functional treatment of baldness Nevertheless; there is general concern that systemic side-effects that may be derived, particularly following topical application of minoxidil and adenosine. Thus it is generally recognized in the medical literature that the side effects of orally administered minoxidil are very serious, and include fluid retention, tachycardia, dyspnea, gynecomastia, fatigue, nausea and cardiotoxicity. There is also evidence that certain side effects have been experienced following topical application of minoxidil. On the other hand, there is a close association of adenosine in cancer formation. The A(1), A(2A), A(2B) and A(3) G-protein-coupled cell surface adenosine receptors (ARs) are found to be upregulated in various tumor cells and activation of the receptors might promote tumor growth via a range of signaling pathways (Fishman et al, 2009).

While various attempts have been made as explained above, current hair tonics have not necessarily had sufficient hair care actions such as prevention of hair loss and promotion of hair growth in any area of the scalps. For examples, finasteride and minoxidil treatments claimed to work on both the vertex area and the frontal area, but are most successful in only the vertex area. In addition, most baldness treatment takes a long period, such as four to six months, before apparently showing the treatment effect. Possibly, the only means which has met with partial success for growing hair on the bald or balding human head is by transplantation of hair to the bald areas. This is usually a painful operation and is not always successful. Furthermore, it is immediately apparent to the casual observer that the subject has received a hair transplant and it may take many months or even years before hair regrowth, following this operation, assumes an appearance which resembles that of the original naturally growing hair.

Current baldness treatments may lift the gloom, but the wide-spread dissatisfactions indicate they are not enough to propel the hair of a relatively regressive and/or resting status toward an active stage, probably due to the diversity of reasons for hair loss and the complexity of the mechanism of hair growth.

It is therefore desirable to provide a novel hair tonic composition having efficient hair care actions on the most areas of scalps.

WO 2008/28773 A1, WO 96/25943 A1, EP 0 107 885 A1 and DE 43 23 616 A1 describe compositions for improving hair growth and for strengthening hair, which include a plurality of active ingredients, for example a combination of purine and bioquinone, of anabolic hormones and minimum essential medium such as adenine, of serum of bovine foetus and nutrients including adenine sulfate or of several nucleic acids.

Starting from the state of the art it is the object of the present application to provide a non-therapeutic use of a composition for promoting hair growth, for promoting eyelash and eyebrow growth and for preventing hair fall, which has a simple texture and an improved effect on hair growth and strengthening.

### SUMMARY OF THE INVENTION

The above-mentioned objects are solved by the non-therapeutic use of a composition for promoting hair growth according to claim 1, the method of promoting eyelash and eyebrow growth according to claim 2, and the non-therapeutic use of a composition for preventing hair fall according to claim 8, respectively. Advantageous improvements of the invention are described by dependent claims.

### BRIEF DESCRIPTION OF THE DRAWINGS

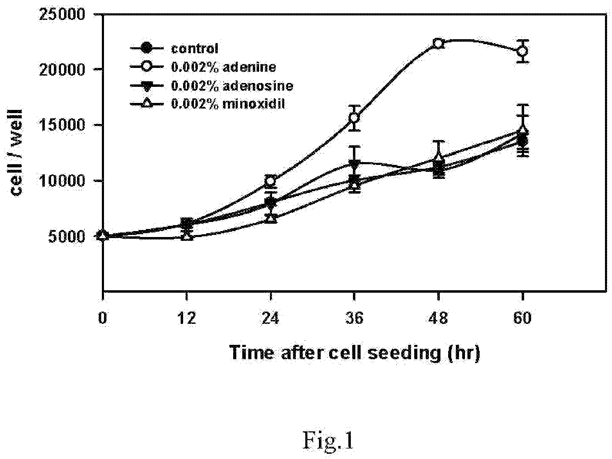

FIG. 1, is a graph showing the results of the promotion of dermal papilla cell proliferation by adenine.

### DESCRIPTION OF THE PREFERRED EMBODIMENTS

The present inventors engaged in broad studies on various substances to solve the problem and found that adenine or salts of adenine have superior hair care actions such as hair loss preventing action and hair growth promoting action, whereby the present invention has been completed.

That is, the present invention provides a non-therapeutic use of a hair tonic composition containing adenine or salts of adenine as only active ingredient. The adenine capable of formulating, as an active ingredient, in the hair tonic composition of the present invention is one type of purine. Further, in the salt of adenine, as the counter ion for forming the salt, any substance may be used so long as forming acid and counter ions. For example, chloride, acetate and sulfate may be mentioned. Further, it is also possible to use the hydrate as the salt of adenine

In the non-therapeutic use of a hair tonic composition of the present invention, adenine or salts of adenine, those which are commercially available as reagents, are used. The content of the adenine and/or salts of adenine in the non-therapeutic use of a hair tonic composition of the present invention can be varied depending upon the form, method of application, of the hair tonic composition of the present invention, preferably 0.001 to 0.1% by weight. If the content is less than 0.001% by weight of the total weight of the hair tonic composition, the desired effect of the present invention does not tend to be sufficiently obtained, and therefore, this is not preferable, while if the amount is more than 0.1% by weight, there is a remarkable tendency of causing problems in the preparation, and therefore is again not preferred.

The type of preparation which the hair tonic composition for the non-therapeutic use of the present invention may take is not particularly limited so long as it is a type of preparation which can be applied to the skin, in particular, the scalp. For example, a liquid, emulsion, ointment may be used. Further, the hair tonic composition for the non-therapeutic use of the present invention may be of any form such as a hair tonic, hair clear lotion, hair care conditioner, hair care gel, hair care mousse and shampoo.

The compositions for the non-therapeutic use of the invention comprise a cosmetically acceptable vehicle to act as a diluant, dispersant or vehicle for adenine, so as to facilitate its distribution when the composition is applied to the skin. Vehicles other than or in addition to water can include liquid or solid emollients, solvents, humectants, thickeners and powders.

### Cosmetically Acceptable Vehicles

The cosmetically acceptable vehicle will usually form from 1% to 99.9%, preferably from 25% to 80% by weight of the composition, and can, in the absence of other cosmetic adjuncts, form the balance of the composition.

The compositions for the non-therapeutic use of the invention may be in the form of aqueous, aqueous/alcoholic or oily solutions; dispersions of the lotion or serum type; anhydrous or lipophilic gels; emulsions of liquid or semiliquid consistency, which are obtained by dispersion of a fatty phase in an aqueous phase (O/W) or conversely (W/O); or suspensions or emulsions of smooth, semi-solid or solid consistency of the cream or gel type. These compositions for the non-therapeutic use of the invention are formulated according to the usual techniques as are well-known to this art.

When the compositions for the non-therapeutic use of the invention are formulated as an emulsion, the proportion of the fatty phase may range from 5% to 80% by weight, and preferably from 5% to 50% by weight, relative to the total weight of the composition. Oils, emulsifiers and co-emulsifiers incorporated in the composition for the non-therapeutic use of the invention in emulsion form are selected from among those used conventionally in the cosmetic or dermatological field. The emulsifer and coemulsifier may be present in the composition for the non-therapeutic use of the invention at a proportion ranging from 0.3% to 30% by weight, and preferably from 0.5% to 20% by weight, relative to the total weight of the composition.

When the compositions for the non-therapeutic use of the invention are formulated as an oily solution or gel, the fatty phase may constitute more than 90% of the total weight of the composition.

The compositions for the non-therapeutic use of the invention may also contain additives and adjuvants which are conventional in the cosmetic, pharmaceutical or dermatological field, such as hydrophilic or lipophilic gelling agents, hydrophilic or lipophilic active agents, preservatives, antioxidants, solvents, fragrances, fillers, bactericides, odor absorbers and dyestuffs or colorants. The amounts of these various additives and adjuvants are those conventionally used in the field, and, for example, range from 0.01% to 10% of the total weight of the composition. Depending on their nature, these additives and adjuvants may be introduced into the fatty phase, into the aqueous phase.

Exemplary oils which may be used according to this invention include plant oils (coconut oil, liquid fraction of karite butter or sunflower oil), mineral oils (liquid petrolatum), animal oils (perhydrosqualene), synthetic oils (purcellin oil), silicone oils (cyclomethicone) and fluoro oils (perfluoropolyethers). Fatty alcohols, fatty acids (stearic acid) and waxes (paraffin wax, carnauba wax and beeswax) may also be used as fats.

Emulsifiers which may be used include glyceryl stearate, polysorbate 60, PEG-6/PEG-32/glycol stearate mixture. Solvents which may be used include the lower alcohols, in particular ethanol and isopropanol, and propylene glycol.

Hydrophilic gelling agents include carboxyvinyl polymers (carbomer), acrylic copolymers such as acrylatelalkylacrylate copolymers, polyacrylamides, polysaccharides, such as hydroxypropylcellulose, natural gums and clays, and, as lipophilic gelling agents, representative are the modified clays such as bentones, fatty acid metal salts such as aluminum stearates and hydrophobic silica, or ethylcellulose and polyethylene.

An oil or oily material may be present, together with an emollient to provide either a water-in- oil emulsion or an oil-in-water emulsion, depending largely on the average hydrophilic-lipophilic balance (HLB) of the emollient employed. Levels of such emollients may range from 0.5% to 50%, preferably between 5% and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1 to 20% by weight, preferably from 0.5% to 10% by weight of the composition. Exemplary thickeners are cross-linked polyacrylate materials available under the trademark Carbopol. Gums may be employed such as xanthan, carrageenan, gelatin, karaya, pectin and locust beans gum. Under certain circumstances the thickening function may be accomplished by a material also serving as a silicone or emollient. For instance, silicone gums in excess of 10 centistokes and esters such as glycerol stearate have dual functionality.

Powders may be incorporated into the cosmetic composition of the invention. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the cosmetic compositions. These ingredients may include coloring agents, opacifiers and perfumes. Amounts of these other adjunct minor components may range anywhere from 0.001% up to 20% by weight of the composition.

The hair tonic composition for the non-therapeutic use of the present invention may be administered transdermally through direct coating or spraying on the skin. Further, the dosage of the hair tonic composition of the present invention differs depending on age, personal differences, states of the disease, form of the hair tonic composition, and cannot be clearly specified, but when administered to humans, the dosage is one by which adenine or salts of adenine are given in an amount of generally 0.0001 to 10.0 mg, preferably 0.001 to 0.1 mg, per kilogram body weight per day. This amount is preferably administered once a day or divided into two to four applications a day.

The hair tonic composition for the non-therapeutic use of the present invention has both a superior hair loss preventing action and hair growth promoting action and hair care action in humans and other mammals and is useful as a pharmaceutical, quasi-pharmaceutical, or cosmetic composition for hair care.

### EXAMPLES

The present invention will now be explained in further detail with reference to, but is not limited to, the following Examples. In the following Examples, the "%" means % by weight, unless otherwise noted.

First, formulations of the hair tonic composition for the non-therapeutic use of the present invention will be given as the following four examples.

### Example 1-4

### Preparation of hair tonic for the non-therapeutic use of the present invention

| | Example 1 | Example 2 | Example 3 | Example 4 | Control |
|---|---|---|---|---|---|
| INGREDIENT | % (w/w) | | | | |
| Adenine sulfate | 0 | 0.0001-0.0005 | 0.001-0.002 | 0.002-0.01 | 0 |
| Folinic acid | 0.0005 | 0.0005 | 0.0001 | 0.0001 | 0.00 01 |
| Menthol | 0.1 | 0.1 | 0.1 | 0.05 | 0.05 |
| Benzoyl peroxide | 0.05 | 0.05 | 0.05 | 0.01 | 0.01 |
| PEG-40 hydrogenated castor oil | 1.0 | 1. | 0.5 | 0.5 | 1.0 |
| Ethanol | 5.0 | 10.0 | 5.0. | 10.0 | 5.0 |
| Ion exchanged water | balance | balance | balance | balance | balance |

### Manufacturing procedure:

(1) Adenine sulfate was mixed and stirred with folinic acid, menthol, benzoyl peroxide, PEG-40 hydrogenated castor oil, ethanol and a part of the ion exchanged water to obtain a solution.
(2) The remainder of the ion exchanged water was then added to the solution to obtain the hair tonic.

Next, the hair growth promoting action of hair tonic Example 1 to 4 obtained above was evaluated. Further, the hair care action of hair tonic Example 3 and 4 obtained above was evaluated.

### Hair Growth Promoting Action Test using animals.

Balb/c mice in the resting stage of the hair cycle were used. That is, six groups of 10 mice each were prepared. The hair was shaved off from the back of the mice by shears and shavers, then hair tonic Example 1 to 4 or the Control were coated on the shaved portions of the mice in the groups once a day in amounts of 0.2 ml a time. The area of re-growth of hair was measured after 14 and 28 days. The results are shown in Table 1 where numerical values are mean values.

**Table 1**

| | Example 1 | Example 2 | Example 3 | Example 4 | Control |
|---|---|---|---|---|---|
| | Area of hair sample re-growth (%) | | | | |
| After 14 days | 5 | 18 | 38 | 65 | 2 |
| After 28 days | 33 | 42 | 82 | 98 | 21 |

As clear from Table 1, the hair tonic composition (hair tonic Example 2, 3 and 4) for the non-therapeutic use of the present invention exhibits a significant effect in the hair growth promoting action test on mice. It was found that the hair tonic composition for the non-therapeutic use of the present invention has a superior hair growth promoting action.

### Hair Care Action Test

The hair care action, including the hair loss preventing action and the hair growth promoting action, of the hair tonic composition for the non-therapeutic use of the present invention was investigated on human subjects by the following method. The test samples were the hair tonic Example 3 and 4, and the Control. That is, the hair tonic Example 3 and 4 and the control were each coated on the scalps of totally 60 male test subjects with the baldness problem twice a day in 1 ml amounts each time for four months straight. The hair care action was evaluated by the assessment of hair characteristics following treatment using questionnaire. The investigating results are summarized in Table 2.

**Table 2**

| | Week 8 | | | Week 16 | | |
|---|---|---|---|---|---|---|
| | Example 3 (n = 25) | Example 4 (n = 28) | Control (n = 17) | Hair tonic 3 (n = 25) | Hair tonic 4 (n = 28) | Control (n = 17) |
| Change of hair appearance | Individuals (%, percentages in the tested group) | | | | | |
| +2 (improvement) | 4 (16%) | 5 (18%) | 0 (0%) | 9 (36%) | 13 (47%) | 0 (0%) |
| +1 (slight improvement) | 7 (28%) | 13 (46%) | 2 (12%) | 10 (40%) | 9 (32%) | 1 (6%) |
| 0 (no change) | 14 (56%) | 10 (36%) | 8 (47%) | 6 (24%) | 6 (21%) | 8 (47%) |
| -1 (slight decline) | 0 (0%) | 0 (0%) | 5 (29%) | 0 (0%) | 0 (0%) | 5 (29%) |
| -2 (decline) | 0 (0%) | 0 (0%) | 2 (12%) | 0 (0%) | 0 (0%) | 3 (18%) |
| Average scoring | 0.60 | 0.82 | -0.41 | 1.12 | 1.25 | -0.59 |

From the results of Table 3, it became clear that hair tonic composition for the non-therapeutic use of the present invention containing adenine or salts of adenine exhibits significant hair care action in human tests.

Further, since a hair growth promoting action was observed even in the hair tonic composition for the non-therapeutic use of the present invention containing adenine (hair tonic Example 3 and 4), it was clear that a similar hair care action is observed even in the hair tonic for the non-therapeutic use of the present invention containing adenine.

### Example 5

### Preparation of hair clear lotion for the non-therapeutic use of the present invention

| INGREDIENT | % (w/w) |
|---|---|
| Phase I | |
| PEG-40 Hydrogenated Castor Oil | 0.5 |
| Tween-20 (Polysorbate 20) | 0.2 |
| Octoxynol 10 (Triton X-100) | 0.1 |
| | |

| Phase II | |
|---|---|
| Adenine sulfate | 0.02 |
| Folinic acid | 0.01 |
| Menthol | 0.1 |
| Benzoyl peroxide | 0.1 |
| Fragrance | 0.2 |
| Ion exchanged water | 98.8 |

### Manufacturing procedure:

(1) Mix well all the components in Phase I and Phase II individually.
(2) Mix the solutions from above to obtain a hair clear lotion.

### Example 6

### Preparation of hair shampoo for the non-therapeutic use of the present invention

| INGREDIENT | % (w/w) |
|---|---|
| Hydroxycellulose | 1.0 |
| Disodium EDTA | 0.1 |
| Sodium citrate | 0.2 |
| Polyquaternium 7 | 2 |
| Sodium laurosyl sarcosinate | 10 |
| lauric acid amidopropylbetaine | 8 |
| Cocamidopropylamine oxide | 6 |
| Lauramide MEA | 6.2 |
| Urea | 0.3 |
| Propyl paraben | 0.2 |
| Adenine sulfate | 0.05 |
| Menthol | 0.1 |
| Benzoyl peroxide | 0.1 |
| Fragrance | 0.2 |
| Ion exchanged water | 65.55 |

### Manufacturing procedure:

(1) Disperse the hydroxycellulose in ion exchanged water by a mixer.
(2) Heat the above solution to 70°C, then add disodium EDTA and sodium citrate until dissolution is complete.
(3) Add Polyquaternium 7 to the above solution until dissolution is complete.
(4) Cool the above solution to 40°C. Add sodium laurosyl sarcosinate, lauric acid amidopropylbetaine, cocamidopropylamine oxide and lauramide MEA and mix well.
(5) Add urea, propyl paraben, adenine sulfate, menthol, benzoyl peroxide and fragrance to obtain the hair shampoo.

### Example 7

### Preparation of hair care conditioner for the non-therapeutic use of the present invention

| INGREDIENT | % (w/w) |
|---|---|
| Polyquaternium 7 | 3.0 |
| Guar hydroxypropyltrimonium chloride | 0.3 |
| Dimethicone copolyol | 1.0 |
| Adenine sulfate | 0.02 |
| Folinic acid | 0.01 |
| Fragrance | 0.5 |
| Ion exchanged water | 95.2 |

### Manufacturing procedure:

(1) Mix ion exchanged water and polyquaternium 7 well, then add guar hydroxypropyltrimonium chloride, dimethicone copolyol, adenine sulfate, folinic acid and fragrance to obtain a hair conditioner.

### Examples 8 and 9

### Preparation of eyelash/eyebrow clear lotion for the method of promoting eyelash and eyebrow growth of the invention

| | Example 8 | Example 9 | Control 2 |
|---|---|---|---|
| INGREDIENT | % (w/w) | | |
| Phase I | | | |
| PEG-40 Hydrogenated Castor Oil | 0.5 | 0.5 | 0.5 |
| Tween-20 (Polysorbate 20) | 0.1 | 0.1 | 0.1 |
| | | | |

| Phase II | | | |
|---|---|---|---|
| Adenine sulfate | 0.1 | 0.05 | 0 |
| Folinic acid | 0.1 | 0.1 | 0.1 |
| Fragrance | 0.2 | 0.2 | 0.2 |
| Pyrogen free water | 99.0 | 99.05 | 99.1 |

### Manufacturing procedure:

(1) Mix well all the components in Phase I and Phase II individually.
(2) Mix the solutions from above to obtain a eyelash lotion

### Eyelashes Growing Test

The eyelashes growing test, of the eyelash clear lotion composition for the method of the present invention was investigated on human subjects by the following method. The test samples were Example 8, Example 9 and the Control 2.

That is, the eyelash clear lotion Example 8, Example 9 and the Control 2 were each coated using cotton swabs on the eyelashes of totally 48 female test subjects once a day in 0.5 ml amounts for three months straight. The eyelashes growing action was evaluated by using questionnaire, as well as measuring the average length of twenty eyelashes (millimeter). The investigating results are summarized in Table 3.

**Table 3**

| | After 2 month application | | |
|---|---|---|---|
| | Example 8 (n = 16) | Example 9 (n = 16) | Control 2 (n = 16) |
| +2 (improvement) | 2 (12.5%) | 4 (25%) | 0 (0%) |
| +1 (slight improvement) | 8 (50%) | 10 (67.5%) | 2 (12.5%) |
| 0 (no change) | 6 (37.5%) | 2 (12.5%) | 12 (75%) |
| -1 (slight decline) | 0 (0%) | 0 (0%) | 2 (12.5%) |
| -2 (decline) | 0 (0%) | 0 (0%) | 0 (0%) |
| Average scoring | 0.75 | 1.13 | 0.13 |
| Average length changing of eyelashes (mm) | 0.12 | 0.22 | 0 |

From the results of Table 3, it became clear that eyelash clear lotion composition for the method of the present invention containing adenine or salts of adenine exhibits significant eyelashes growing action in human tests.

### Eyebrow Growing Test

The eyebrow growing test, of the eyebrow clear lotion composition for the method of the present invention was investigated on human subjects by the following method. The test samples were Example 8, Example 9 and the Control 2.

That is, the eyebrow clear lotion Example 8, Example 9 and the Control 2 were each coated using cotton swabs on the eyebrows of totally 60 test subjects once a day in 0.5 ml amounts for two months straight. The eyebrow growing action was evaluated by using questionnaire, as well as measuring the average length of ten eyebrows (millimeter). The investigating results are summarized in Table 4.

**Table 4**

| | After 2 month application | | |
|---|---|---|---|
| | Example 8 (n = 20) | Example 9 (n = 20) | Control 2 (n = 20) |
| +2 (improvement) | 4 (20%) | 3 (15%) | 0 (0%) |
| +1 (slight improvement) | 6 (30%) | 10 (50%) | 2 (10%) |
| 0 (no change) | 10 (50%) | 7 (35%) | 18 (90%) |
| -1 (slight decline) | 0 (0%) | 0 (0%) | 0 (0%) |
| -2 (decline) | 0 (0%) | 0 (0%) | 0 (0%) |
| Average scoring | 0.7 | 0.8 | 0.1 |
| Average length changing of eyelashes (mm) | 0.18 | 0.25 | 0 |

From the results of Table 4, it became clear that eyebrow clear lotion composition for the method of the present invention containing adenine or salts of adenine exhibits significant eyebrow growing action in human tests.

### Human dermal papilla cell proliferation test

The promotion of cell proliferation by adenine was evaluated using human dermal papilla cells, compared with adenosine, and minoxidil under the following conditions, the results are shown in FIG. 1.

Determination of the proliferation of human dermal papilla cells in the culture media containing adenine, adenosine, and minoxidil. Supplements: 0.002% of adenine, adenosine, minoxidil.

### Test method:

(1) Prepare 0.002% adenine, adenosine, minoxidil containing testing media using the hair follicle dermal papilla cell media (cat. No. 611-500, Cell applications, Inc) .
(2) Seed the isolated human dermal papilla cells in 96-well culture plate with a density of 5,000 cells per well. Add 0.1 mL prepared culture media into individual well.
(3) At 12, 24, 36, 48 and 60 hour after cell seeding, visually count the total cell number using hemacytometer.
(4) Evaluate the result.

From the results of Fig.1, it became apparent that adenine promotes the proliferation of human dermal papilla cells. As explained above, according to the present invention, there is provided a hair tonic composition for the non-therapeutic use of the present invention having superior hair care actions such as a hair loss preventing action and hair growth promoting action in humans and other mammals.

## Claims

1. A non-therapeutic use of a composition for promoting hair growth in a human for applying to scalp or hair roots of said human, the composition comprising as the sole active ingredient effective on promoting hair growth an effective amount of adenine or salts of adenine.

2. A method of promoting eyelash and eyebrow growth in a human, comprising applying a composition on the eyelash/eyebrow of said human, wherein the composition comprising as the sole active ingredient effective on promoting eyelash and eyebrow growth an effective amount of adenine or salts of adenine.

3. The use of claim 1, wherein an effective amount of adenine or salts of adenine is combined with a cosmetically acceptable vehicle.

4. The method of claim 2, wherein an effective amount of adenine or salts of adenine is combined with a cosmetically acceptable vehicle.

5. The use of claim 1, wherein the effective amount of the active ingredient is 0.0001% to 10% by weight, based upon the total weight of the composition.

6. The use of claim 3, wherein the vehicle is selected from the group consisting of liquid or solid emollients, solvents, humectants, thickeners and powders.

7. The method of claim 4, wherein the vehicle is selected from the group consisting of liquid or solid emollients, solvents, humectants, thickeners and powders.

8. A non-therapeutic use of a composition for preventing hair fall in a human for applying to scalp or hair roots of said human, the composition comprising as the sole active ingredient effective on preventing hair fall an effective amount of adenine or salts of adenine.

9. The use of claim 1 or 8, wherein the composition is a hair tonic or hair clear lotion or hair care conditioner or hair care gel or hair care mousse or shampoo.

10. The method of claim 2, wherein the composition is eyelash clear lotion or eyebrow clear lotion.

## Patentansprüche

1. Nicht-therapeutische Verwendung einer Zusammensetzung zur Unterstützung des Haarwachstums bei einem Menschen zum Auftragen auf die Kopfhaut oder die Haarwurzeln des Menschen, wobei die Zusammensetzung als einzigen aktiven Inhaltsstoff, der bei der Unterstützung des Haarwachstums wirksam ist, eine wirksame Menge an Adenin oder Adeninsalzen umfasst.

2. Verfahren zur Unterstützung des Wachstums von Wimpern und Augenbrauen bei einem Menschen, umfassend das Auftragen einer Zusammensetzung auf die Wimpern/Augenbrauen des Menschen, wobei die Zusammensetzung als einzigen aktiven Inhaltsstoff, der bei der Unterstützung des Wachstums von Wimpern und Augenbrauen wirksam ist, eine wirksame Menge an Adenin oder Adeninsalzen umfasst.

3. Verwendung nach Anspruch 1, wobei eine wirksame Menge an Adenin oder Adeninsalzen mit einem kosmetisch akzeptablen Trägerstoff kombiniert ist.

4. Verfahren nach Anspruch 2, wobei eine wirksame Menge an Adenin oder Adeninsalzen mit einem kosmetisch akzeptablen Trägerstoff kombiniert wird.

5. Verwendung von Anspruch 1, wobei die wirksame Menge des aktiven Inhaltsstoffs 0,0001 Gew.-% bis 10 Gew.-% bezogen auf das Gesamtgewicht der Zusammensetzung beträgt.

6. Verwendung nach Anspruch 3, wobei der Trägerstoff ausgewählt ist aus der Gruppe bestehend aus flüssigen oder festen Weichmachern, Lösungsmitteln, Feuchthaltemitteln, Verdickungsmitteln und Pulvern.

7. Verfahren nach Anspruch 4, wobei der Trägerstoff ausgewählt ist aus der Gruppe bestehend aus flüssigen oder festen Weichmachern, Lösungsmitteln, Feuchthaltemitteln, Verdickungsmitteln und Pulvern.

8. Nicht-therapeutische Verwendung einer Zusammensetzung zur Verhinderung von Haarausfall bei einem Menschen zum Auftragen auf die Kopfhaut oder die Haarwurzeln des Menschen, wobei die Zusammensetzung als einzigen aktiven Inhaltsstoff, der bei der Verhinderung von Haarausfall wirksam ist, eine wirksame Menge an Adenin oder Adeninsalzen umfasst.

9. Verwendung nach Anspruch 1 oder 8, wobei die Zusammensetzung ein Haarwasser oder eine klare Haarlotion oder ein Haarpflegemittel oder ein Haarpflegegel oder ein Haarpflegeschaum oder -shampoo ist.

10. Verfahren nach Anspruch 2, wobei die Zusammensetzung eine klare Wimpernlotion oder eine klare Augenbrauenlotion ist.

## Revendications

1. Utilisation non thérapeutique d'une composition permettant de favoriser la croissance des cheveux chez un humain pour application au cuir chevelu ou aux racines des cheveux dudit humain, la composition comprenant, en tant que le seul ingrédient actif efficace pour favoriser la croissance des cheveux, une quantité efficace d'adénine ou de sels d'adénine.

2. Procédé permettant de favoriser la croissance des cils et sourcils chez un humain, comprenant l'application d'une composition sur les cils/sourcils dudit humain, la composition comprenant, en tant que le seul ingrédient actif efficace pour favoriser la croissance des cils et sourcils, une quantité efficace d'adénine ou de sels d'adénine.

3. L'utilisation de la revendication 1, sachant qu'une quantité efficace d'adénine ou de sels d'adénine est combinée à un vecteur cosmétiquement acceptable.

4. Le procédé de la revendication 2, sachant qu'une quantité efficace d'adénine ou de sels d'adénine est combinée à un vecteur cosmétiquement acceptable.

5. L'utilisation de la revendication 1, sachant que la quantité efficace de l'ingrédient actif est de 0,0001 % à 10 % en poids, sur la base du poids total de la composition.

6. L'utilisation de la revendication 3, sachant que le vecteur est sélectionné dans le groupe constitué par des émollients, solvants, humectants, épaississants et poudres liquides ou solides.

7. Le procédé de la revendication 4, sachant que le vecteur est sélectionné dans le groupe constitué par des émollients, solvants, humectants, épaississants et poudres liquides ou solides.

8. Utilisation non thérapeutique d'une composition permettant d'empêcher la chute des cheveux chez un humain pour application au cuir chevelu ou aux racines des cheveux dudit humain, la composition comprenant, en tant que le seul ingrédient actif efficace pour empêcher la chute des cheveux, une quantité efficace d'adénine ou de sels d'adénine.

9. L'utilisation de la revendication 1 ou 8, sachant que la composition est une lotion tonique pour cheveux ou une lotion claire pour cheveux ou un revitalisant de soins pour cheveux ou un gel de soins pour cheveux ou une mousse ou un shampoing de soins pour cheveux.

10. Le procédé de la revendication 2, sachant que la composition est une lotion claire pour cils ou une lotion claire pour sourcils.
